# EUROPEAN PATENT APPLICATION

(11) **EP 4 112 745 A1**
(43) Date of publication of application: **04.01.2023**
(21) Application number: 21775124.7
(22) Date of filing: 24.03.2021
(51) Int. Cl.: C12Q 1/6883, G01N 33/50

(54) **BIOMARKERS FOR PREDICTING A PATIENT'S RESPONSE TO BCG THERAPY, METHODS AND USES BASED THEREON**

(30) Priority: 25.03.2020 ES 202030244
(71) Applicant: Fundación para la Investigación Biomédica del Hospital Universitario 12 de Octubre, 28041 Madrid (ES); Centro de Investigaciones Energéticas, Medioambientales y Tecnológicas O.A., M.P. (CIEMAT), 28040 Madrid (ES)
(72) Inventor: DUEÑAS PORTO, Marta Gloria, 28029 Madrid (ES); SUÁREZ CABRERA, Cristian, 28039 Madrid (ES); PARAMIO GONZÁLEZ, Jesús María, 28045 Madrid (ES); GUERRERO RAMOS, Félix, 28045 Madrid (ES)
(74) Representative: Manuel Illescas y Asociados, S.L.
(86) International application number: PCT/ES2021/070204
(87) International publication number: WO 2021/191485

(57) **Abstract**

The present invention relates to an *in vitro* method for predicting the response of a bladder cancer patient to Bacillus Calmette-Guerin (BCG) immunotherapy, comprising determining the expression level or ratio of expression levels of miRNAs or combinations of miRNAs in a sample and comparing said expression level, or ratio of expression levels, to a reference value, wherein a difference in said comparison is indicative of said patient's response to BCG therapy. The present invention also relates to kits for carrying out such methods and to uses of miRNAs, or combinations of miRNAs, to predict whether or not a bladder cancer patient will respond to BCG therapy.

## Description

### TECHNICAL FIELD OF THE INVENTION

The present invention comprises biomarkers, specifically microRNAs (miRNAs) or combinations of miRNAs related to methods for predicting the response of a subject with bladder cancer to a therapy with *Bacillus Calmette Guerin* (BCG), based on the expression levels of said biomarkers and with kits for carrying out such methods.

### BACKGROUND OF THE INVENTION

Bladder cancer is the most common malignant neoplasm of the urinary tract, being a highly prevalent disease that mainly affects older people. It is the 11th most common cancer diagnosed worldwide and a major cause of tumour-related mortality, with approximately 150,000 deaths per year. Bladder cancer can be divided into two major classes based on tumour stage, (i) non-muscle-invasive bladder cancer (NMIBC), which is confined to the urothelium (carcinoma *in situ* (CIS), Ta stage) or lamina propria (T1 stage); and (ii) muscle-invasive bladder cancer (MICC) (T2, T3, and T4 stages).

NMIBC is the most common form of bladder cancer, being found in 70-80% of patients at the time of diagnosis and is mainly treated by transurethral resection of bladder tumour (TURBT). Patients at increased risk of recurrence are treated, after resection, with serial intravesical instillations with *Bacillus Calmette Guerin* (BCG), an attenuated strain of *Mycobacterium bovis* that has been used as a tuberculosis vaccine and has been shown to be effective against bladder cancer.

Kiselyov et al. described cellular and molecular markers relevant to the immune response triggered by BCG instillation in patients with non-muscle-invasive bladder cancer (NMIBC), and mathematical models of treatment of NMIBC with BCG (Kiselyov et al, 2015).

Treatment with BCG presents considerable side effects that seriously affect the quality of life of patients, such as severe inflammation, cystitis, urethritis, pelvic pain, fever, joint pain, lower back pain and pain when urinating, so there exists, in the state of the art, the need to develop a precise method of predicting response to BCG, which avoids applying BCG to a patient who does not respond to such therapy, with the consequent benefit of preventing the serious side effects associated with BCG therapy.

### DESCRIPTION OF THE INVENTION

As used herein and in the claims, singular forms such as "the" include references to plural forms unless the content clearly indicates otherwise.

Throughout the description and the claims, the term "comprising", "that comprises" and their variants are meant in a non-limiting sense and therefore should not exclude other technical features. The term "comprises", "comprising" and their variants, throughout the description and claims, specifically includes the term "consists of", "consisting of" and their variants.

Unless defined otherwise, all the technical and scientific terms used throughout the description and claims have the same meaning as those customarily understood by a person skilled in the field of the invention.

In the present patent, "microRNA" or "miRNA" refer to non-coding RNA molecules between, generally, 20 and 25 nucleotides in length, found in various organisms, including animals. It is usual in the state of the art to name miRNAs using the prefix "miR" and a unique identification number. As an exception, the terms lin-4 and let-7 are also used to name the first miRNAs identified, originally in the organism *Caenorhabditis elegans,* and which have maintained their name ever since. The mature miRNA (the gene expression product) is designated, for example, as miR-21, whereas mir-21 refers to the gene encoding said mature miRNA. It is usual in the state of the art to include an additional three-letter prefix, which refers to the organism. Thus, hsa-miR-21 refers to human miR-21 (hsa refers to *Homo sapiens*)*.* All miRNAs of the present invention are human miRNAs, e.g., miR-21, in the context of the present invention, refers to hsa-miR-21. The presence of letters after the miRNA identification number refers to closely related mature sequences; for example, hsa-miR-121a and hsa-miR-121b refer to closely related mature sequences. The term "5p" and "3p" at the end of the miRNA name refers to the sequence of the 5' or 3' arm, respectively. Mature miRNA sequences and gene sequences encoding such mature miRNA sequences are generally available in accessible databases. Examples of accessible databases specializing in RNA sequences are miRBase (http://www.mirbase.org/) and RNACentral (https://rnacentral.org/).

In the present patent, the term "predict" refers to evaluating the probability that a subject will respond to the treatment to which this patent refers. As is commonly understood by those skilled in the art, generally, such evaluation does not intend to be correct for 100% of the subjects to be evaluated. The term, however, requires that the response to such therapy of a statistically significant portion of the subjects can be predicted. Whether a portion is statistically significant can be determined by using various statistical evaluation tools that are well known in the art, such as determining confidence intervals and determining the p-value, for example, via binomial testing. Preferred confidence intervals are at least 90%, at least 95%, at least 97%, at least 98%, or at least 99%. Preferably, the probability predicted by the present invention enables the prediction to be correct for at least 60%, at least 70%, at least 80%, or at least 90% of the subjects in a given cohort or population. Preferably, the prediction method has a sufficiently large sensitivity and specificity, as described below. Preferably, the sensitivity provided by the present invention enables the prediction to be correct for at least 75%, at least 80%, at least 85%, at least 90%, or at least 95% of subjects in a given cohort or population.

In the present patent, *"p-value"* refers to a probability value, ranging from 0 to 1 and representing a measure of statistical significance. The p-value is the probability that a calculated statistical value is possible, given a true null hypothesis. The p-value helps to differentiate results that are the product of randomness from results that are statistically significant. For a particular result, if the p-value is less than an arbitrarily defined value, it is considered as a statistically significant result and enables the null hypothesis to be rejected. Preferably, said value is 0.1, 0.05, 0.01, 0.005 or 0.0001.

In the present patent, the accuracy of a prediction can be obtained by applying standard statistical methods. In particular, such a prediction can be obtained by Receiver-Operating Characteristics (*ROC*). The ROC graph is a graph of all sensitivity versus specificity pairs, resulting from continuously varying the decision threshold over the entire range of observed data. The clinical performance of a prediction method depends on its accuracy, that is, its ability to correctly assign subjects to a given prognosis or diagnosis. The ROC graph indicates the overlap between the two distributions by plotting sensitivity against the value 1 - specificity value for the full range of cut-off values suitable for making a distinction. On the y-axis is the sensitivity, or the ratio (or %) of true positives, which is defined as the ratio between the number of true positives and the sum of the number of true positives and the number of false negatives. On the x-axis is the ratio of false positives, or 1 - specificity (or 100% - specificity %), which is defined as the ratio between the number of false positives and the sum of the number of true negatives and the number of false positives. Each point on the ROC chart represents a sensitivity/specificity pair corresponding to a particular cut-off value. A test with perfect discrimination (no overlap in the two result distributions) has a ROC graph that passes through the upper left corner, where the ratio of true positives is 1.0, or 100% (perfect sensitivity) and the ratio of false positives is 0 (perfect specificity). The theoretical diagram for a non-discriminatory test (identical distributions of results for the two groups) is a 45° diagonal line from the lower left corner to the upper right corner. Most graphs lie between these two extremes. Qualitatively, the closer the graph to the top left corner, the higher the overall accuracy of the test. Depending on a desired confidence interval, a cut-off value can be derived from the ROC graph that enables the prediction of a given event with an appropriate balance of sensitivity and specificity, respectively. Accordingly, the reference to be used for the methods of the present invention may preferably be generated by establishing a ROC for said cohort as described above and deriving a cut-off value therefrom. Depending on the sensitivity and specificity desired for a prediction method, the ROC graph enables the obtaining of suitable cut-off values. Preferably, the reference amounts are within the range of values representing a sensitivity of at least 75% and a specificity of at least 45%, or a sensitivity of at least 80% and a specificity of at least 40%, or a sensitivity of at least 85% and a specificity of at least 33%, or a sensitivity of at least 90% and a specificity of at least 25%.

In the present patent, "AUC" refers to the value under the curve in a ROC graph. Higher AUC values indicate better method performance and possible AUC values range from 0.5 (no predictive capability) to 1.0 (perfect predictive capability).

In the present patent, the term "subject" refers to animals, preferably mammals, and, more preferably, humans. More preferably, said subject has had in the past, or has in the present, or is suspected to have, or is at risk of having, bladder cancer. Even more preferably, said subject has had bladder cancer and is, or is suspected to be, at risk of recurrence of bladder cancer. Subjects who are affected by said disease may be identified by the symptoms accompanying the disease, which are known in the state of the art. However, a subject suspected of being affected by the aforementioned disease may also be an apparently healthy subject, for example, investigated by routine clinical examination, or may be a subject at risk of developing the aforementioned disease. The groups at risk for the disease are known in the state of the art. The groups at risk of recurrence of bladder cancer are known in the state of the art.

In the present patent, *"Bacillus Calmette-Guerin* (BCG) therapy" refers to an anticancer therapy that has been proven effective against bladder cancer. BCG is an attenuated strain of *Mycobacterium bovis* that has been used as a tuberculosis vaccine. It was first used to treat bladder cancer in 1976. BCG is a potent stimulator of host defence mechanisms and can induce tumour regression in immunocompetent hosts. Repeated instillation of BCG produces a non-specific immune reaction within the bladder that enables the dislodging of cells from the bladder tissue, especially those cancerous cells that divide rapidly. BCG is usually administered after superficial bladder tumours have been removed during an operation. BCG is administered intravesically through a catheter that is placed in the bladder, and is administered repeatedly over a period of time. BCG has emerged as an effective intravesical treatment for superficial bladder cancer and carcinoma *in situ* (CIS). The effects of BCG on bladder cancer are only local and there is no protection against the development of tumours in areas where there is no contact with the BCG.

In the present patent, the "expression level" of a miRNA in a sample from a subject can be determined by techniques well known in the art. Depending on the nature of the sample, the amount of a miRNA can be determined by PCR-based techniques to quantify the amount of a polynucleotide or by other methods such as mass spectrometry or sequencing, or one of the methods described in the examples.

In the present patent, the "ratio of expression levels" is obtained by means of a mathematical operation, such as division or multiplication, between the expression levels of the miRNAs within a combination of miRNAs, or between a variable derived from said expression levels, such as the Ct value. Preferably, said combination is a pair. Preferably, the ratio of expression levels of a pair of miRNAs is determined by dividing the expression level of a first miRNA of the pair of miRNAs by the expression level of the second miRNA of the pair of miRNAs.

Herein, the term "sample" refers to a sample of a body fluid; to a sample of cells; to a sample of a tissue, or of an organ; or to a wash/rinse fluid sample obtained from an external or internal body surface. The samples can be obtained by means of well-known techniques, and preferably include biopsies. More preferably, the samples are samples of body fluids, e.g., preferably, blood, plasma, serum, urine, saliva, tears, and fluids obtainable from the mammary glands, e.g., milk. More preferably, the samples are urine samples. More preferably, the bodily fluid samples are free of cells from the subject. Tissue or organ samples may be obtained from any tissue or organ by means of, for example, a biopsy or other surgical procedures. More preferably, the tissue samples are bladder tissue samples. Cells may be obtained from the body fluids or from tissues or organs by separation techniques such as filtration, centrifugation, or cell sorting. Preferably, the samples of cells, tissues or organs are obtained from body fluids, cells, tissues or organs known or suspected to contain the miRNAs of the present invention. More preferably, the samples are obtained from those body fluids, cells, tissues or organs containing the miRNAs of the invention described below in this patent.

In the present patent, the "expression level" of a miRNA can be determined by techniques well known in the art, including, but not limited to, RT-qPCR, DNA microarray-based methods, and the nCounter^{®} analysis system of NanoString Technologies, Inc.

In the present patent, "RT-qPCR" means real-time quantitative polymerase chain reaction (PCR) and refers to a technique of quantifying the number of RNA transcripts corresponding to a particular gene present in samples. In particular, it can be used to determine gene expression in cells and tissues. As in conventional PCR, it employs a DNA template, at least one specific primer pair, dNTPs, a suitable reaction buffer, and a thermostable DNA polymerase. A substance labelled with a fluorophore is added to said mixture, enabling the measurement of the rate of generation of one or more specific products in a thermocycler equipped with fluorescence sensors, after excitation of the fluorophore at the appropriate wavelength. This measurement is performed after each amplification cycle. In many cases, the template used for quantitative PCR is not DNA, but can be complementary DNA (cDNA), single stranded, obtained by retrotranscription of ribonucleic acid (RNA). Quantification can be performed in absolute or relative terms. In the first case, the strategy is to relate the amplification signal obtained to the DNA content using a calibration curve. In the second case, the change in messenger RNA (mRNA) expression levels is expressed using complementary DNA obtained by retrotranscription of the mRNA. Relative quantification does not require a calibration curve and is based on the comparison between the level of expression of the gene to be studied, with respect to a reference gene (also called control, normalizer or housekeeping gene). Different methods are known in the art for evaluating RT-qPCR results, one of which is the ΔCt method, in which "Ct" means cycle threshold values. During the RT-qPCR reaction, the number of cycles of each sample in which the fluorescence crosses an arbitrary line (PCR amplification indicator), the cycle threshold, is measured. This cross-point is the Ct value. To quantify the gene expression of a particular gene, the Ct of a nucleic acid from the gene of interest is divided by the Ct of the nucleic acid from the reference gene, in the same sample, to normalize the variation in the amount of RNA between different samples, and to obtain the relative expression of the sample with respect to the reference gene.

The nCounter^{®} analysis system of NanoString Technologies, Inc. uses pairs of specific probes for certain genes that hybridize directly to the mRNA in solution and avoids using retrotranscription or amplification reactions. In the first step of the assay, the DNA probes hybridize directly to a region of 70-100 base pairs of the mRNA in solution. The fluorescent labelling probe consists of a sequence of 35-50 base pairs complementary to the target mRNA molecule and a single DNA sequence that hybridizes to six RNA segments labelled with one of four differently coloured fluorescent probes. The fluorescent probes create a six-position, four-colour "colour code," unique to each target molecule. A capture probe consisting of biotin and a sequence of 35-50 base pairs, complementary to the target mRNA molecule, is used, enabling the immobilization of the probe/target complexes on a slide with a layer of streptavidin. After hybridization, the sample purification steps are performed on an automatic platform. First, the excess capture and labelling probes are eliminated by successive capture steps using magnetic spheres, followed by bonding the probe/target complexes to random points on the surface of a cartridge, via the streptavidin-biotin bond. Finally, the probe/target complexes are aligned and immobilized in the cartridge. The cartridge is then placed in a digital analyser for data collection. Each target molecule of interest is identified by the "colour code" present in the associated labelling probe. At that time, the labelling probes on the surface of the cartridge corresponding to each target molecule are counted and the data are analysed.

In the present patent, the term "compare" refers to contrasting the expression level or ratio of expression levels of miRNAs or miRNA pairs, referred to in the present patent, in the sample to be analysed with an expression level or ratio of expression levels of said miRNA or miRNA pair in a suitable reference sample, as specified below in the present description. The comparison refers to that of the corresponding parameters or values, for example, an absolute amount of miRNA is compared to an absolute reference amount of said miRNA; a concentration of miRNA is compared to a reference concentration of said miRNA; an intensity signal obtained from the miRNA in a sample is compared to the same type of intensity signal from said miRNA in a reference sample. The above comparison can be carried out manually or may be computer-assisted. In the case of a computer-assisted comparison, the value of the particular quantity can be compared with the values corresponding to the appropriate references that are stored in a database by means of a computer program. Accordingly, the result of the identification referred to in this document may be provided automatically in an appropriate output format.

In the present patent, the term "reference value" is derived from samples from subjects obtained after BCG therapy, by means of which it is known whether or not the subjects from whom such samples are derived respond to BCG therapy. This reference value may be a discrete figure or may be a range of values. Evidently, the reference value may vary between individual miRNA species. Thus, the measurement system is preferably calibrated with a sample or with a series of samples comprising known amounts of each specific miRNA. The applicable reference value for an individual subject may vary, depending on various physiological parameters such as age, or subpopulation. Thus, a suitable reference value can be determined, by means of the methods of the present invention, from a reference sample, to be analysed together, i.e. simultaneously, or subsequently, with the test sample. Further, preferably, a cut-off value may be used as a reference value. Preferably, a reference value may be derived from a sample from a subject, or group of subjects, who are known to respond to BCG therapy. Preferably, a reference value can also be derived from a sample of a subject or group of subjects, which is known not to respond to BCG therapy. It should be understood that the above values may vary, due to statistics and measurement errors. Reference values can be calculated for a group, or cohort of subjects, as specified herein, based on average values for a given miRNA, by applying standard statistical methods.

In the present patent, the term "difference in comparison" refers to a decrease or increase in the expression level, or ratio of expression levels of miRNAs, or pairs of miRNAs, referred to in the present patent, in the sample to be analysed, relative to an expression level, or expression levels, of said miRNA, or pair of miRNAs, in a suitable reference sample, as specified above. Preferably, said difference is statistically significant, i.e., a statistically significant decrease, or a statistically significant increase. Whether a difference is statistically significant can be determined, using various statistical evaluation tools, well known in the art, e.g., determination of confidence intervals and determination of the p-value, e.g., via binomial testing. Preferred confidence intervals are at least 90%, at least 95%, at least 97%, at least 98%, or at least 99%. The significance levels of the statistical tests are preferably 0.1, 0.05, 0.01, 0.005 or 0.0001.

In the present patent, the term "indicative" refers to the fact that a difference between the expression level, or ratio of expression levels of miRNAs, or pairs of miRNAs, mentioned in the present patent, in the sample to be analysed, with respect to an expression level or expression levels of said miRNA or pair of miRNAs, in a suitable reference sample, makes it possible to predict whether a subject responds to the BCG treatment to which this patent refers.

The technical problem to be solved consists of obtaining an improved, high-precision BCG response prediction method that avoids applying BCG to a patient who does not respond to such therapy, with the consequent benefit of preventing the serious side effects associated with BCG therapy.

This invention, defined by the claims, provides a solution to said technical problem.

The present invention provides an improved, highly accurate BCG response prediction method.

The examples demonstrate that the BCG response prediction method is a highly accurate method.

In the present invention, miRNA pairs were identified that could be used to distinguish BCG-responding patients from non-responding patients. Such miRNA pairs of the invention are: miR-21/miR-182, miR-222/miR-182, miR-429/miR-182, miR223/miR-182, and miR-21/miR-106a.

Additionally, differentially expressed miRNAs were identified in the tumours of patients who showed a satisfactory response in contrast to those who presented recurrence of the tumour, over a period of less than three years. Said miRNAs of the invention are: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342 and miR-99a.

The present invention provides an *in vitro* method for predicting the response of a subject with bladder cancer to a therapy with *Bacillus Calmette-Guerin* (BCG), comprising determining, in a sample obtained from said subject, the expression level or ratio of expression levels of:
(i) at least one pair of miRNAs selected from the group consisting of: miR-21/miR-182, miR-222/miR-182, miR-429/miR-182, miR223/miR-182, and miR-21/miR-106a; or combinations of such pairs; or
(ii) at least one miRNA selected from the group consisting of: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, and miR-99a, or combinations thereof; and comparing said expression level or ratio of expression levels with a reference value.

The method of the invention, based on the miRNAs, or combinations of miRNAs of the invention described above, predicts which patients will display a favourable response to treatment by intravesical instillations with BCG. That is, it predicts which patients will benefit from this type of therapy from those who, despite receiving this treatment, will show a recurrence of the disease.

The method of the invention has substantial advantages, such as avoiding applying BCG to a patient who does not respond to such therapy, with the consequent benefit of preventing the serious side effects associated with BCG therapy, such as severe inflammation, cystitis, urethritis, pelvic pain, fever, joint pain, lower back pain and pain when urinating.

The miRNAs and combinations of miRNAs of the invention can be detected in any sample from a subject. Preferably, such miRNAs and combinations of miRNAs are detected in tissue or urine samples. More preferably, said urine samples are prior to the surgery before BCG treatment.

In a preferred embodiment of the method of the invention, said method comprises determining, in a sample obtained from said subject, the expression level or ratio of expression levels of a miRNA, or a pair of miRNAs, selected from the group consisting of: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342 and miR-99a, miR-21/miR-182, miR-222/miR-182, miR-429/miR-182, miR223/miR-182 and miR-21/miR-106a.

In a more preferred embodiment of the method of the invention, said method comprises determining, in a sample obtained from said subject, the level of expression or ratio of levels of expression of a miRNA, or a pair of miRNAs, selected from the group consisting of: miR-21-5p, miR-106a-5p, miR-17-5p, miR-222-3p, miR-429, miR-223-3p, let-7c-5p, miR-29a-3p, miR-199a-3p, miR-199b-3p, miR-199b-5p, miR-23a-3p, miR-218-5p, miR-125b-5p, miR-23b-3p, miR-199a-5p, miR-1-3p, miR-483-3p, miR-145-5p, miR-4443, miR-874-5p, miR-497-5p, miR-143-3p, miR-574-3p, miR-579-3p, miR-342-3p, miR-99a-5p, miR-21-5p/miR-182-5p, miR-222-3p/miR-182-5p, miR-429/miR-182-5p, miR223-3p/miR-182-5p and miR-21-5p/miR-106a-5p.

In one preferred embodiment of the method of the invention, the sample is an urine tissue sample.

In a more preferred embodiment of the method of the invention, the tissue sample is a bladder cancer tumour tissue sample.

In a preferred embodiment of the method of the invention, the combination of miRNAs is miR-21/miR-106a.

In a preferred embodiment of the method of the invention, the ratio of the expression levels of at least one pair of miRNAs selected from the group consisting of: miR-21/miR-182, miR-222/miR-182, miR-429/miR-182, and miR223/miR-182 is determined, the sample being a tissue sample.

In a preferred embodiment of the method of the invention, the ratio of the expression levels of at least one pair of miRNAs selected from between miR-21/miR-182 or miR-21/miR-106a is determined, the sample being urine.

In a preferred embodiment of the method of the invention, said miRNA is selected from: miR-106a, or miR-21, the sample being urine.

The present invention likewise provides the in *vitro* use of a biomarker selected from the group consisting of:
(i) at least one pair of miRNAs selected from among: miR-21/miR-182, miR-222/miR-182, miR-429/miR-182, miR223/miR-182, and miR-21/miR-106a; or combinations of said pairs; or
(ii) miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, and miR-99a; or combinations thereof;
to predict the response of a subject with bladder cancer to a therapy with *Bacillus Calmette-Guerin* (BCG), wherein the expression level, or ratio of expression levels of said biomarker, in a sample obtained from said subject, is indicative of the response of said subject to BCG therapy.

In a preferred embodiment of the use of the invention, said biomarker is a miRNA or pair of miRNAs, selected from the group consisting of: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, miR-99a, miR-21/miR-182, miR-222/miR-182, miR-429/miR-182 and miR-223/miR-182.

In one embodiment of the method of the invention, the sample is a tissue sample or urine sample.

In a more preferred embodiment of the use of the invention, the tissue sample is a bladder cancer tumour tissue sample.

In a preferred embodiment of the use of the invention, the biomarker is the pair of miRNAs: miR-21/miR-106a.

In a preferred embodiment of the use of the invention, said biomarker is selected from the group of miRNA pairs consisting of: miR-21/miR-182, miR-222/miR-182, miR-429/miR-182 and miR223/miR-182, the sample being a tissue sample.

In a preferred embodiment of the use of the invention, said biomarker is selected from the miRNA pairs consisting of: miR-21/miR-182, or miR-21/miR-106a, the sample being urine.

In a preferred embodiment of the use of the invention, said biomarker is selected from: miR-21 or miR-106a, the sample being urine.

The present invention also provides a kit for predicting the response of a subject with bladder cancer to a therapy with *Bacillus Calmette-Guerin* (BCG), comprising the reagents necessary to determine, in a sample obtained from said subject, the expression level or ratio of expression levels of:
(i) at least one biomarker selected from the group of miRNA pairs consisting of: miR-21/miR-182, miR-222/miR-182, miR-429/miR-182, miR223/miR-182, and miR-21/miR-106a; or combinations of such pairs; or
(ii) at least one biomarker selected from the group consisting of: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, and miR-99a; or combinations thereof.

In a preferred embodiment of the kit of the invention, said biomarker is a miRNA, or pair of miRNAs, selected from the group consisting of: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, miR-99a, miR-21/miR-182, miR-222/miR-182, miR-429/miR-182 and miR223/miR-182.

In a preferred embodiment of the kit of the invention, the sample tested in said kit is a tissue sample or urine sample.

In a more preferred embodiment of the kit of the invention, the tissue sample tested in said kit is a bladder cancer tumour tissue sample.

In a preferred embodiment of the kit of the invention, the biomarker is the pair of miRNAs: miR-21/miR-106a.

In a preferred embodiment of the kit of the invention, said biomarker is selected from the group of miRNA pairs consisting of: miR-21/miR-182, miR-222/miR-182, miR-429/miR-182 and miR223/miR-182, the sample tested in said kit being a tissue sample.

In a preferred embodiment of the kit of the invention, said biomarker is selected from the miRNA pairs consisting of: miR-21/miR-182 or miR-21/miR-106a, the sample tested in said kit being urine.

In a preferred embodiment of the kit of the invention, said biomarker is selected from: miR-21 or miR-106a, the sample tested in said kit being urine.

In a preferred embodiment of the use or kit of the invention, said biomarker is a miRNA or pair of miRNAs, selected from the group consisting of: miR-21-5p, miR-106a-5p, miR-17-5p, miR-222-3p, miR-429, miR-223-3p, let-7c-5p, miR-29a-3p, miR-199a-3p, miR-199b-3p, miR-199b-5p, miR-23a-3p, miR-218-5p, miR-125b-5p, miR-23b-3p, miR-199a-5p, miR-1-3p, miR-483-3p, miR-145-5p, miR-4443, miR-874-5p, miR-497-5p, miR-143-3p, miR-574-3p, miR-579-3p, miR-342-3p, miR-99a-5p, miR-21-5p/miR-182-5p, miR-222-3p/miR-182-5p, miR-429/miR-182-5p, miR223-3p/miR-182-5p and miR-21-5p/miR-106a-5p.

### BRIEF DESCRIPTION OF THE DRAWINGS

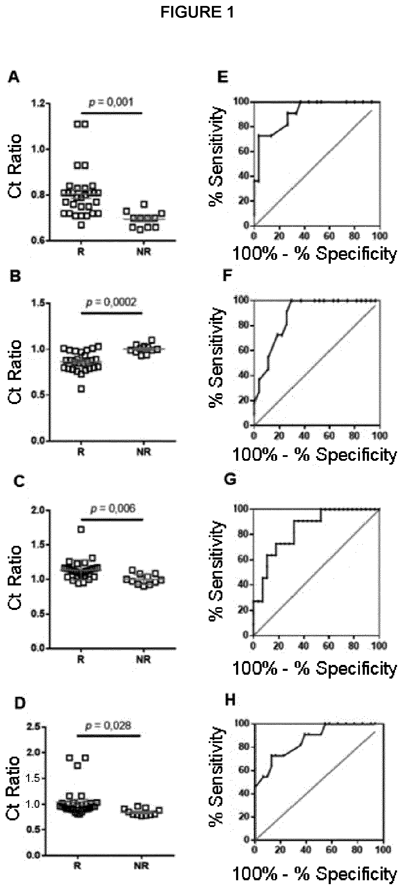

Figure 1. Representation of the Ct value ratios of the miR-21-5p/miR-182-5p (A), miR-222-3p/miR-182-5p (B), miR223-3p/miR-182-5p (C) and miR-429/miR-182-5p (D) pairs among BCG-responding (R) and non-responding (NR) patients. The mean of each group and its standard deviation are shown. ROC curves for the relationships of expression levels of the miR-21-5p/miR-182-5p (E), miR-222-3p/miR-182-5p (F), miR223-3p/miR-182-5p (G) and miR-429/miR-182-5p (H) pairs, obtained from the results shown in graphs A-D. The AUC values of the ROC curves were as follows: 0.90 (E), 0.88 (F), 0.84 (G) and 0.87 (H).

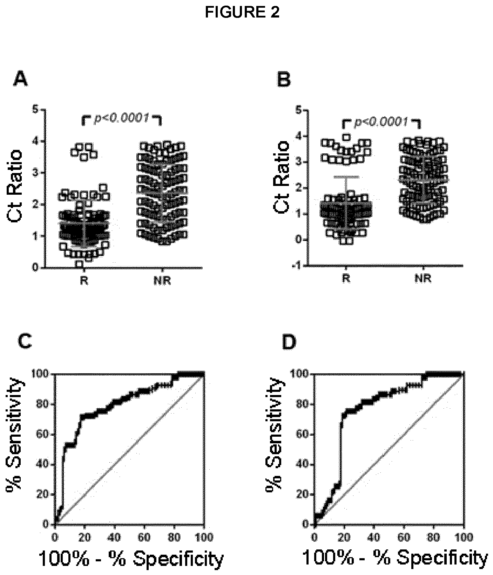

Figure 2. Representation of the Ct value ratios of the miR-21-5p/miR-182-5p (A) and miR-21-5p/miR-106a-5p (B) pairs among BCG-responding (R) and non-responding (NR) patients. The mean of each group and its standard deviation are shown. ROC curves for the relationships of expression levels of the miR-21-5p/miR-182-5p (C) and miR-21-5p/miR-106a-5p (D) pairs, obtained from the results shown in graphs A-B. The AUC values of the ROC curves obtained were 0.79 (C) and 0.76 (D).

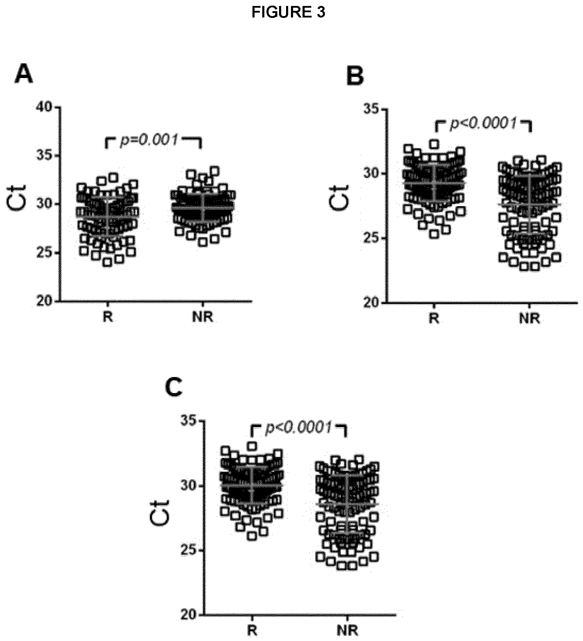

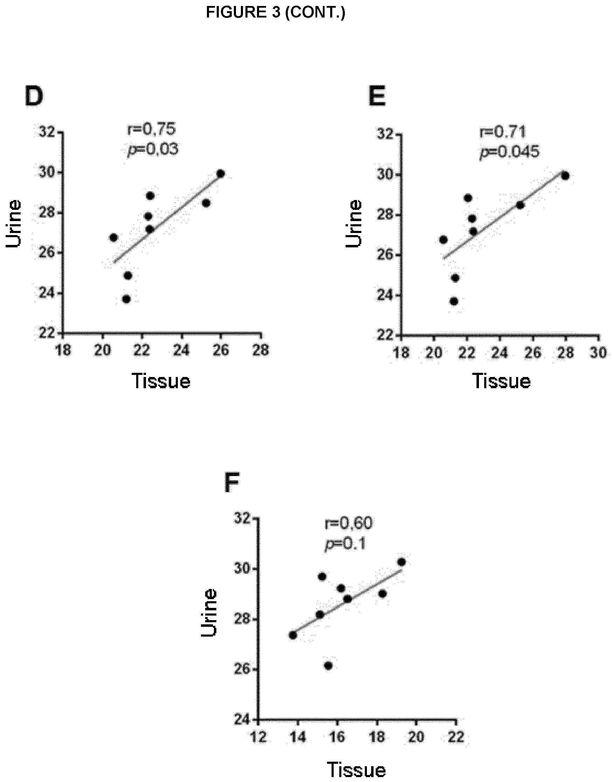

Figure 3. Representation of Ct values of miR-182-5p (A), miR-106a-5p (B) and miR-21-5p (C) among BCG-responding (R) and non-responding (NR) patients. The mean of each group and its standard deviation are shown. Correlation between Ct values of miR-182-5p (D), miR-106a-5p (E) and miR-21-5p (F) in tissue and urine.

### DESCRIPTION OF EMBODIMENTS

### Patient Cohorts

Samples from three patient cohorts from the "12 de Octubre" Hospital in Madrid, Spain, were used: discovery cohort, validation cohort and test cohort.

Table 1 shows the clinical-pathological characteristics and parameters of patients in the discovery cohort, which consisted of 39 patients (25 responders and 14 non-responders to BCG therapy). Primary paraffinised tumour tissue samples from these patients were used, taken at the time of transurethral resection of the bladder tumour (TURBT) and subsequently subjected to BCG instillation therapy.

**Table 1**

| | |
|---|---|
| Patients (Male; Female) | 39 (M = 34; F = 5) |
| Responders - Non-Responders | 25 - 14 |
| Mean Age and Range | 69 (49-80). |
| Tumour Stage | Ta = 13; T1 = 25; Tx = 1 |
| Tumour grade | G1 = 8: G2 = 31 |

| | |
|---|---|
| Tx = tumour stage not determined | |

Table 2 shows the clinical-pathological characteristics and parameters of the patients in the validation cohort, which consisted of 43 patients (28 responders and 15 non-responders to BCG therapy). Primary paraffinised tumour tissue samples from these patients were used, taken at the time of transurethral resection of the bladder tumour (TURBT) and subsequently subjected to BCG instillation therapy.

**Table 2**

| | |
|---|---|
| Patients (Male; Female) | 43 (M = 36; F = 7) |
| Responders - Non-Responders | 28 - 15 |
| Mean Age and Range | 65 (53-80). |
| Tumour Stage | Ta = 19; T1 = 24 |
| Tumour grade | G1 = 8: G2 = 35 |

Table 3 shows the clinical-pathological characteristics and parameters of the patients in the test cohort, which consisted of 20 patients (11 responders and 9 non-responders to BCG therapy). Urine samples from these patients were used, obtained prior to transurethral resection of the bladder tumour (TURBT), and who subsequently underwent BCG instillation therapy.

**Table 3**

| | |
|---|---|
| Patients (Male; Female) | 20 (M = 19; F = 1) |
| Responders - Non-Responders | 11 -9 |
| Mean Age and Range | 59 (52-85). |
| Tumour Stage | CIS = 3; Ta = 7; T1 = 8; Tx = 2 |
| Tumour grade | G1= 1; G2 = 6; G3= 10; Gx= 3 |

| | |
|---|---|
| Tx = tumour stage not determined; Gx = tumour grade not determined. | |

### Example 1. miRNAs differentially expressed in tissue samples from responding and non-responding patients to BCG therapy

In paraffinised tumour tissue samples from the 39 BCG-treated bladder cancer patients in the discovery cohort (25 responders and 14 non-responders to BCG therapy), miRNA expression was detected by the digital nCounter Human v3 miRNA panel assay (NanoString Technologies, Inc.). Said paraffinised primary tumour tissue samples from patients in the discovery cohort were taken at the time of transurethral resection of the bladder tumour (TURBT) and subsequently underwent BCG instillation therapy.

A statistical analysis of the results of the digital assay was performed. In this statistical analysis, 26 differentially expressed miRNAs (7 induced and 19 repressed) were identified between responder and non-responder patients to BCG therapy (Table 4). These 26 miRNAs were differentially expressed in the tumours of the patients who showed a satisfactory response with respect to those who presented recurrence of the tumour in a period of less than 3 years.

Relative increase expression values between responder and non-responder patients to BCG therapy are shown in Table 4. These relative increase expression values between responder and non-responder patients to BCG therapy range from +20.37 (for miR-579-3p) to -7.28 (for miR-1-3p).

Additionally, the expression of the 26 miRNAs was detected by RT-qPCR, and a statistical analysis of the data obtained with both assays was performed. Table 4 shows the p-value obtained in the statistical analysis between responders (R) and non-responders (NR) obtained in the digital assay and in the RT-qPCR assay.

**Table 4**

| miRNA | Relative increase R vs NR | P-value (NanoString digital assay) | P-value (RT-qPCR assay) |
|---|---|---|---|
| let-7c-5p | -2.11 | 0.00109 | 0.013 |
| miR-29a-3p | -2.05 | 0.00230 | 0.050 |
| miR-222-3p | -1.74 | 0.00517 | 0.015 |
| miR-199a-3p/miR-199b-3p * | -2.28 | 0.00601 | 0.039 |
| miR-23a-3p | -1.72 | 0.00642 | 0.032 |
| miR-223-3p | -4.16 | 0.00942 | 0.006 |
| miR-199b-5p | -2.26 | 0.01039 | 0.033 |
| miR-218-5p | -3.20 | 0.01091 | NS |
| miR-125b-5p | -3.34 | 0.01321 | NS |
| miR-23b-3p | -1.77 | 0.01353 | 0.033 |
| miR-199a-5p | -1.84 | 0.01443 | 0.041 |
| miR-1-3p | -7.28 | 0.01735 | 0.048 |
| **miR-483-3p** | 6.72 | 0.01885 | 0.025 |
| miR-145-5p | -3.12 | 0.02327 | 0.030 |
| **miR-106a-5p **** | 2.06 | 0.02724 | 0.036 |
| miR-4443 | 1.79 | 0.02726 | 0.024 |
| **miR-874-5p** | 3.37 | 0.02841 | 0.025 |
| miR-497-5p | -2.24 | 0.03099 | NS |
| miR-143-3p | -3.14 | 0.03452 | 0.046 |
| miR-574-3p | -2.91 | 0.03465 | NS |
| **miR-579-3p** | 20.37 | 0.03616 | 0.027 |
| miR-342-3p | -1.65 | 0.03671 | NS |
| **miR-182-5p** | 2.03 | 0.03870 | 0.042 |
| miR-21-5p | -2.34 | 0.03730 | 0.023 |
| **miR-429** | 2.24 | 0.04484 | 0.050 |
| miR-99a-5p | -5.30 | 0.04592 | 0.047 |

In bold, induced miRNAs, the remainder of miRNAs are repressed miRNAs. NS: not significant.
* The sequences of said miRNAs are identical
** In the Nanostring assay, said miRNA and miR-17-5p, which only differs from miR-106a-5p in one nucleotide, are indistinguishable.

Table 5 shows the correspondence between the sequences of the sequence listing and the mature sequences of the miRNAs.

**Table 5**

| miRNA | SEQ ID NO |
|---|---|
| let-7c-5p | 1 |
| miR-29a-3p | 2 |
| miR-222-3p | 3 |
| miR-199a-3p/miR-199b-3p | 4 |
| miR-23a-3p | 5 |
| miR-223-3p | 6 |
| miR-199b-5p | 7 |
| miR-218-5p | 8 |
| miR-125b-5p | 9 |
| miR-23b-3p | 10 |
| miR-199a-5p | 11 |
| miR-1-3p | 12 |
| miR-483-3p | 13 |
| miR-145-5p | 14 |
| miR-106a-5p | 15 |
| miR-4443 | 16 |
| miR-874-5p | 17 |
| miR-497-5p | 18 |
| miR-143-3p | 19 |
| miR-574-3p | 20 |
| miR-579-3p | 21 |
| miR-342-3p | 22 |
| miR-182-5p | 23 |
| miR-21-5p | 24 |
| miR-429 | 25 |
| miR-99a-5p | 26 |
| miR-17-5p | 27 |

### Example 2. Pairs of miRNAs in predicting response to BCG therapy

In paraffinised tumour tissue samples from the 43 BCG-treated bladder cancer patients in the validation cohort (28 responders and 15 non-responders to BCG therapy), Ct values were obtained by RT-qPCR from each of the 26 miRNAs in Table 4. Said primary paraffinised tumour tissue samples from patients in the validation cohort were taken at the time of transurethral resection of the bladder tumour (TURBT) and who subsequently underwent BCG instillation therapy.

Statistical analysis was performed to evaluate the capability of pairs of the 26 miRNAs in Table 4 of differentiating between samples corresponding to BCG responder and non-responder patients. Surprisingly, the miR-21-5p/miR-182-5p, miR-222-3p/miR-182-5p, miR-429/miR-182-5p, and miR223-3p/miR-182-5p pairs differentiated between BCG-responder and non-responder patients with a very high statistical significance and very high AUC values. Figure 1 shows the values of the Ct ratios, obtained by dividing the Ct value of the miRNA that decreases by the Ct value of the miRNA that increases. The Ct value is inversely proportional to the amount of miRNA detected in the sample and, consequently, the values of the Ct ratios obtained are positive. The AUC values obtained ranged from 0.84 for the miR223-3p/miR-182-5p pair to 0.90 for the miR-21-5p/miR-182-5p pair (Figure 1).

### Example 3. Evaluation of miRNAs and miRNA pairs in predicting response to BCG therapy in urine samples

An evaluation was made of the ability of the miR-21-5p, miR-182-5p and miR-106a-5p biomarkers and the miR-21-5p/miR-182-5p and miR-21-5p/miR-106a-5p pairs to differentiate between samples corresponding to BCG responders and non-responders in urine samples from the 20 test cohort patients (11 responders and 9 non-responders to BCG therapy), which had been obtained prior to transurethral resection of the bladder tumour (TURBT) in said patients, who subsequently underwent BCG instillation therapy.

A statistical analysis was performed to assess the capability of said biomarkers to differentiate between samples corresponding to BCG-responder and non-responder patients, in urine samples.

Surprisingly, the miR-21-5p/miR-106a-5p and miR-21-5p/miR-182-5p pairs differentiated responders from non-responders to BCG therapy with a high statistical significance and high AUC values. The AUC values obtained were 0.79 for the miR-21-5p/miR-106a-5p pair and 0.76 for the miR-21-5p/miR-182-5p pair (Figure 2), indicating that said miRNA pairs are good predictors of bladder cancer patients' response to BCG therapy.

The Ct expression values of miR-21-5p, miR-182-5p and miR-106a-5p differentiated responders from non-responders to BCG therapy in urine samples, with a high statistical significance (Figure 3A-C). Therefore, miRNAs miR-21-5p and miR-106a-5p are good predictors of the response of bladder cancer patients to BCG therapy. The expression values of miR-21-5p and miR-106a-5p showed a good correlation with the levels obtained in tumour tissue (Figure 3D-F).

### REFERENCES

Kiselyov et al. (2015). Treatment of non-muscle invasive bladder cancer with Bacillus Calmette-Guerin (BCG): Biological markers and simulation studies. BBA Clin., 10(4), 27-34.

## Claims

1. An *in vitro* method for predicting the response of a subject with bladder cancer to a therapy with *Bacillus Calmette-Guerin* (BCG), comprising determining in a sample obtained from said subject the expression level or ratio of expression levels of:
(i) at least one pair of miRNAs selected from the group consisting of: miR-21/miR-182, miR-222/miR-182, miR-429/miR-182, miR223/miR-182, and miR-21/miR-106a; or combinations of said pairs; or
(ii) at least one miRNA selected from the group consisting of: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, and miR-99a; or combinations thereof; and
comparing said expression level, or ratio of expression levels, with a reference value, wherein a difference in said comparison, is indicative of said subject's response to BCG therapy.

2. The method according to claim 1, wherein the sample is a tissue sample, or urine sample.

3. The method according to claim 2, wherein the tissue sample is a bladder cancer tumour tissue sample.

4. The method according to any one of claims 1 to 3, wherein the ratio of expression levels of at least one pair of miRNAS selected from: miR-21/miR-182, miR-222/miR-182, miR-429/miR-182, and miR223/miR-182 is determined; the sample being a tissue sample.

5. The method according to any one of claims 1 to 3, wherein the ratio of expression levels of at least one pair of miRNAs selected from: miR-21/miR-182, or miR-21/miR-106a is determined; the sample being urine.

6. The method according to any one of claims 1 to 3, wherein the level of expression of a miRNA selected from: miR-21 or miR-106a is determined; the sample being urine.

7. *In vitro* use of a biomarker selected from the group consisting of:
(i) at least one pair of miRNAs selected from among: miR-21/miR-182, miR-222/miR-182, miR-429/miR-182, miR223/miR-182, and miR-21/miR-106a; or combinations of said pairs; or
(ii) miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, and miR-99a; or combinations thereof;
to predict the response of a subject with bladder cancer to a therapy with *Bacillus Calmette-Guerin* (BCG), wherein the expression level, or ratio of expression levels of said biomarker, in a sample obtained from said subject, is indicative of the response of said subject to BCG therapy.

8. The use according to claim 7, wherein the sample is a tissue sample or urine sample.

9. The use according to claim 8, wherein the tissue sample is a bladder cancer tumour tissue sample.

10. The use according to any one of claims 7 to 9, wherein said biomarker is selected from the group of miRNA pairs consisting of: miR-21/miR-182, miR-222/miR-182, miR-429/miR-182 and miR223/miR-182, the sample being a tissue sample.

11. The use according to any one of claims 7 to 9, wherein said biomarker is selected from the miRNA pairs: miR-21/miR-182, or miR-21/miR-106a, the sample being urine.

12. The use according to any one of claims 7 to 9, wherein said biomarker is selected from: miR-21, or miR-106a, the sample being urine.

13. A kit for predicting the response of a subject with bladder cancer to a therapy with *Bacillus Calmette-Guerin* (BCG), comprising the reagents necessary to determine, in a sample obtained from said subject, the expression level or ratio of expression levels of:
(i) at least one biomarker selected from the group of miRNA pairs consisting of: miR-21/miR-182, miR-222/miR-182, miR-429/miR-182, miR223/miR-182, and miR-21/miR-106a; or combinations of said pairs; or
(ii) at least one biomarker selected from the group consisting of: miR-21, miR-106a, miR-17, miR-222, miR-429, miR-223, let-7c, miR-29a, miR-199a, miR-199b, miR-23a, miR-218, miR-125b, miR-23b, miR-1, miR-483, miR-145, miR-4443, miR-874, miR-497, miR-143, miR-574, miR-579, miR-342, and miR-99a; or combinations thereof.

14. The kit according to claim 13, wherein the sample tested in said kit is a tissue sample or urine sample.

15. The kit according to claim 14, wherein the tissue sample tested in said kit is a bladder cancer tumour tissue sample.

16. The kit according to any one of claims 13 to 15, wherein said biomarker is selected from the group of miRNA pairs consisting of: miR-21/miR-182, miR-222/miR-182, miR-429/miR-182 and miR223/miR-182; the sample tested in said kit being a tissue sample.

17. The kit according to any one of claims 13 to 15, wherein said biomarker is selected from the miRNA pairs: miR-21/miR-182 or miR-21/miR-106a; the sample tested in said kit being urine.

18. The kit according to any one of claims 13 to 15, wherein said biomarker is selected from: miR-106a or miR-21; the sample tested in said kit being urine.
